# EUROPÄISCHE PATENTSCHRIFT

(11) **EP 2 767 264 B1**
(45) Veröffentlichungstag und Bekanntmachung des Hinweises auf die Patenterteilung: **23.08.2017**
(21) Anmeldenummer: 14154435.3
(22) Anmeldetag: 10.02.2014
(51) Int. Cl.: A61F 2/966, A61F 2/95

(54) **Abdeckvorrichtung zum Abdecken eines zumindest teilweise freigegebenen medizinischen Implantats in einem Katheter und Katheter mit einer Abdeckvorrichtung**
Cover device for covering an at least partly released medical implant in a catheter, and catheter comprising a cover device
Dispositif de recouvrement destiné à recouvrir un implant médical au moins partiellement découvert, dans un cathéter, cathéter doté d'un dispositif de recouvrement

(30) Priorität: 13.02.2013 US 201361763973 P
(43) Veröffentlichungstag der Anmeldung: 20.08.2014
(73) Patentinhaber: Biotronik AG, 8180 Bülach (CH)
(72) Erfinder: Trajkovic, Biljana, 8157 Dielsdorf (CH); Pantic, Dragica, 8051 Zürich (CH); Fargahi, Amir, 8180 Bülach (CH)
(74) Vertreter: Galander, Marcus

(56) Entgegenhaltungen:
- EP-A2- 2 789 313
- US-A1- 2010 049 313
- US-A1- 2011 245 917

## Beschreibung

Die Erfindung betrifft eine Abdeckvorrichtung zum Abdecken eines zumindest teilweise freigegebenen medizinischen Implantats in einem Katheter und Katheter mit einer Abdeckvorrichtung zum Abdecken eines zumindest teilweise freigegebenen medizinischen Implantats bei einer Implantation in einen tierischen und/oder menschlichen Körper.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina Implantate, zahnmedizinische Implantate, Implantate zum Gelenkersatz, Gefäßprotesen oder Stents. Auf dem Feld der Herzimplantate sind beispielsweise Klappenimplantate, wie etwa Aortenklappenimplantate, bekannt, welche die Funktion der natürlichen Aortenklappe übernehmen. Hierbei wird das Klappenimplantat nach Expansion der Implantatstruktur sofort nach der Implantation fixiert und nimmt die Position der natürlichen Aortenklappe ein.

Implantate werden vor einem Einführen in den Körper mit Kathetern verbunden und müssen so befestigt sein, dass sie am Einsatzort komplikationslos vom Katheter genau platziert und definiert freigegeben werden können. Ein häufiges Problem ist hierbei, dass das Implantat mit einer Fehlstellung fixiert wird, was zu einem Versagen des Implantats führen kann. Hierzu ist beispielsweise bekannt, einen Außenschaft des Katheters wieder über ein teilweise freigegebenes Implantat zu schieben, wodurch dieses in seinen ursprünglichen Durchmesser zusammengedrückt wird. Im Anschluss erfolgt eine erneute Implantation.

US 2011/0245917 offenbart eine Einführvorrichtung zur Implantation einer stentbasierten Herzklappenprothese.

Der Erfindung liegt die Aufgabe zugrunde, eine Abdeckvorrichtung anzugeben, mit der ein Abdecken eines Implantats mit einer Einführvorrichtung einfach und bedienerfreundlich möglich ist und mit der über beispielsweise einer Repositionierung eines Implantats ein hochpräzises und gezieltes Freigeben eines Implantats erfolgen kann.

Eine weitere Aufgabe ist in der Bereitstellung einer entsprechenden Einführvorrichtung zu sehen.

Eine zudem weitere Aufgabe kann darin gesehen werden, ein Verfahren zum Abdecken des teilweise freigegebenen medizinischen Implantats mit Hilfe der erfindungsgemäßen Abdeckvorrichtung bereitstellen, bei dem ein Einführen, ein Abdecken, eine Repositionierung und eine Freigabe des medizinischen Implantats, schnell, zuverlässig und komplikationslos erfolgen kann.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird eine Abdeckvorrichtung zum Abdecken eines zumindest teilweise freigegebenen medizinischen Implantats vorgeschlagen, welches von einer Einführvorrichtung durch eine Relativbewegung zwischen einem ersten und einem zweiten Einführelement freisetzbar ist, umfassend zumindest einen Expansionskörper, an dem eine erste radiale Erstreckung und zumindest eine zweite radiale Erstreckung einstellbar ist, wobei die zweite radiale Erstreckung des zumindest einen Expansionskörpers bei dem Abdecken des zumindest teilweise freigegebenen medizinischen Implantats an zumindest eine radiale Erstreckung des zumindest teilweise freigegebenen medizinischen Implantats anpassbar ist, wobei der zumindest eine Expansionskörpers zumindest in wenigstens einem axialen Abschnitt in Umfangsrichtung im Wesentlichen homogen aufweitbar ist.

Durch die erfindungsgemäße Ausgestaltung kann eine Abdeckvorrichtung bereitgestellt werden, mit der eine Abdeckung des zumindest teilweise freigegebenen Implantats (im folgenden Text freigegebenes Implantat genannt) schnell, komplikationsarm und schonend erfolgen kann. Dadurch kann auch eine Dauer eines Eingriffs an einem Patienten für diesen vorteilhaft verkürzt werden. Besonders vorteilhaft kann mittels der erfindungsgemäßen Ausgestaltungen eine Wiedereinhüllung bzw. ein so genanntes Resheathing und damit eine Repositionierung eines fehlplatzierten freigegebenen Implantats ermöglicht werden. Dies ist insbesondere der Fall, da sich der zumindest eine Expansionskörper (im folgenden Text auch lediglich als der Expansionskörper bezeichnet) flexibel an eine Kontur des Implantats anpassen kann. Zudem kann ein Funktionstest des Implantats und im Defektfall eine Entfernung des missfunktionalen Implantats komfortabel unterstützt durch den Expansionskörper erfolgen. Des Weiteren kann bei einem Einsatz eines solchen Expansionskörpers eine Einführvorrichtung, wie beispielsweise ein Katheter, verwendet werden, das besonders kompakt ausgestaltet ist. Trotz einer Ausführung des Expansionskörpers mit einer zweiten Erstreckung, welche auf eine Erstreckung des freigegebenen Implantats angepasst ist, ist somit eine geringe, insbesondere, radiale Erstreckung der Einführelemente der Einführvorrichtung realisierbar. Damit weist die Einführvorrichtung gegenüber Systemen des Standes der Technik vorteilhaft einen geringeren Durchmesser auf. Zusätzlich kann durch den Einsatz des Expansionskörpers ein Risiko einer Deformation des Implantats sowie einer daraus resultierende Blockade der Einführvorrichtung eliminiert werden. Als ein weiterer Vorteil kann genannt werden, dass das Konzept des Expansionskörpers auf viele verschiedene Einführvorrichtungen und Implantate anwendbar ist.

In diesem Zusammenhang soll unter einem Implantat ein Körper verstanden werden, der zumindest eine Ersatzfunktion permanent oder für einen längeren Zeitraum bei Implantation in einen tierischen und/oder menschlichen Körper erfüllt. Denkbar wären hier sämtliche, dem Fachmann als zweckdienlich erscheinende medizinische Implantate, wie beispielsweise ein Herzschrittmacher, ein Hirnschrittmacher, ein Herzimplantat, ein Cochleaimplantat, ein Retina Implantat, ein zahnmedizinisches Implantat, ein Implantat zum Gelenkersatz, eine Gefäßprothese, eine Appendixprothese oder besonders vorteilhaft wird eine Ausbildung des medizinischen Implantats als ein Klappenimplantat vorgeschlagen. Unter einem "Klappenimplantat" soll insbesondere ein Körper verstanden werden, der zumindest eine Ersatzfunktion eines Rückschlagventils, permanent oder für einen längeren Zeitraum bei Implantation erfüllt. Denkbar wären hier sämtliche, dem Fachmann als zweckdienlich erscheinende medizinische Klappenimplantate, wie beispielsweise ein Aortenklappen-, ein Pulmonalklappen-, ein Mitralklappen- oder ein Trikuspidalklappenimplantat.

Unter einem Expansionskörper soll hier ein Körper verstanden werden, der sich passiv und/oder aktiv an eine Form, wie bspw. die Form eines anderen Körpers, z.B. einen Bereich eines Implantats, anpassen kann. Hierbei soll unter Anpassung sowohl eine Expansion bzw. eine Ausdehnung, wie auch eine entgegengesetzt zu der Expansion wirkende Aktion bzw. eine Verringerung verstanden werden. Die Anpassung kann hier von dem Expansionskörper selbst oder von außerhalb des Expansionskörpers, wie beispielsweise durch eine Änderung eines Drucks, einer Temperatur, eines pH-Werts, einer Konzentration eines Proteins, eines Ions, eines Salzes etc. oder durch Anlegen einer Spannung, ausgelöst werden. Die radialen Erstreckungen stellen hier beispielsweise Durchmesser dar. Die erste Erstreckung ist bevorzugt kleiner als die zweite Erstreckung. Zudem kann an dem Expansionskörper eine Vielzahl von unterschiedlichen radialen Erstreckungen einstellbar sein. Ferner können die erste und/oder die zweite radiale Erstreckung lediglich an einem Bereich des Expansionskörpers einstellbar sein. Unter der Wendung "die zweite radiale Erstreckung des zumindest einen Expansionskörpers [... ist] an zumindest eine radiale Erstreckung des zumindest teilweise freigegebenen medizinischen Implantats anpassbar" soll verstanden werden, dass die zweite radiale Erstreckung sich im Wesentlichen an die Erstreckung eines expandierten Bereichs des Implantats anpasst, wobei "im Wesentlichen" bedeutet, dass eine Abweichung der Erstreckungen von bis zu 30% als angepasst gilt.

Ferner soll in diesem Zusammenhang unter "zumindest in wenigstens einem axialen Abschnitt in Umfangsrichtung im Wesentlichen homogen aufweitbar" verstanden werden, dass zumindest ein Bereich, der sich in axialer Richtung bzw. in Längsrichtung des Expansionskörpers erstreckt, über seinen gesamten Umfang im Wesentlichen gleichmäßig aufweitet. In anderen Worten, exemplarisch für eine runde Ausgestaltung des Expansionskörpers ausgedrückt, ein Änderung des radialen Abstands von Punkten, die entlang des Umfangs des Expansionskörpers auf derselben axialen Höhe angeordnet sind, von einer Mittelachse des Expansionskörpers ist für alle Punkte im Wesentlichen dieselbe. Unter im Wesentlichen sollen hier auch Abweichungen der Aufweitungen bzw. der Expansionsstrecken der Punkte von bis zu 10% verstanden werden.

In einer bevorzugten Ausgestaltung der Abdeckvorrichtung weist der zumindest eine Expansionskörper zumindest bei eingestellter zweiter radialer Erstreckung eine zu einer Innenachse der Einführvorrichtung wirkenden Rückstellkraft auf, die dazu ausgelegt ist, ein Rückexpandieren des zumindest teilweise freigegebenen medizinischen Implantats auszulösen. Hierdurch kann das Rückexpandieren des Implantats konstruktiv einfach anhand einer im Expansionskörper vorhandenen Kraft bewerkstelligt werden, wodurch Platz und Bauraum eingespart werden können, da ein separates Rückstellmittel entfallen kann. Die Rückstellkraft kann beispielsweise eine Radialkraft und/oder eine Federkraft sein. Alternativ und/oder zusätzlich kann der Expansionskörper in Bereichen auch elastisch ausgeführt sein. Günstigerweise weist der Abschnitt des Expansionskörpers, an dem die zumindest zweite radiale Erstreckung eingestellt ist die zu der Innenachse der Einführvorrichtung wirkenden Rückstellkraft auf.

Eine besonders flexibel und vielseitig einsetzbare Abdeckvorrichtung kann bereitgestellt werden, wenn die zumindest zweite radiale Erstreckung des zumindest einen Expansionskörpers bezüglich der ersten radialen Erstreckung des zumindest einen Expansionskörpers bevorzugt zweimal, vorteilhaft dreimal und besonders bevorzugt mehr als fünfmal größer ist. Es hat sich beispielsweise gezeigt, dass Ausgestaltungen des Expansionskörpers gewählt werden können, bei denen die erste radiale Erstreckung bspw. 4 mm und die zumindest zweite radiale Erstreckung z. B. 25 mm beträgt. Es ergibt sich somit eine 6,25-fache Steigerung der Erstreckung oder in anderen Worten ein Anstieg um 525%.

Zudem kann es vorteilhaft sein, wenn der zumindest eine Expansionskörper zumindest ein Geflecht aufweist, wodurch sich der Expansionskörper besonders einfach und flexibel an die radiale Erstreckung des Implantats anpassen kann. Des Weiteren kann der Expansionskörper so insbesondere bei einem gebogenen Zustand, wie bei einem Durchführen der Einführvorrichtung entlang eines Aortenbogens, eine glatte und regelmäßige Form beibehalten und/oder annehmen. Der Expansionskörper weist also keine Haken, scharfe spitzige Kanten und/oder Schlitze auf. Damit wird das Risiko einer Verletzung von passierten Geweben und/oder Organen patientenfreundlich reduziert und/oder beseitigt. Der Expansionskörper weist somit zumindest einen so genannten Braid auf und/oder ist als Braid ausgeführt. Hierbei soll unter einem Geflecht eine Struktur mit mehreren ineinandergeschlungenen Strängen aus biegsamem Material verstanden werden. Dieses Material kann von jedem, dem Fachmann für einsetzbar erachtetem Material gebildet sein, wie einem weichen Metall, einem flexiblen Kunststoff und/oder einem Textilen Werkstoff. Zudem wäre auch eine Kombination von mehreren Materialien denkbar. Dies könnte entweder ein Mischmaterial sein oder einzelne Stränge könnten aus unterschiedlichen Materialien gefertigt sein. Vorteilhafterweise kann der zumindest eine Expansionskörper aus Elgiloy gefertigt sein, wodurch dieser korrosionsbeständig ist, insbesondere gegen Körperflüssigkeiten, und eine hohe Festigkeit, Dehnbarkeit, und gute Haltbarkeit aufweist.

In einer bevorzugten Realisierung ist das Geflecht reversibel faltbar ausgeführt, um die erste radiale Erstreckung und die zumindest zweite radiale Erstreckung einzustellen. Unter "reversibel faltbar" soll hier verstanden werden, dass das Geflecht zumindest zwei Zustände (erste und zweite radiale Erstreckung) einnehmen kann, zwischen denen es durch einen Faltprozess und/oder Klappprozess, wie Ausklappen und/oder Einklappen, wechseln kann. Durch diese Ausgestaltung können die Zustandsänderungen einfach und schnell erfolgen. Zudem kann so konstruktiv einfach eine große Änderung in der radialen Erstreckung des Expansionskörpers realisiert werden. In einer weiteren Ausgestaltung der Erfindung wird vorgeschlagen, dass das zumindest eine Geflecht zumindest ein Metallgeflecht aufweisen und/oder als ein Metallgeflecht ausgebildet sein kann. Ein so ausgestalteter Expansionskörper bzw. Braid hat sehr gute Schiebe- und Führungseigenschaften, wodurch dieses besonders komfortabel manipuliert werden kann.

Vorteilhafterweise und bevorzugt kann das zumindest eine Geflecht aus ineinander verflochtenem Runddraht gefertigt sein, dadurch kann der Faltprozess vorteilhaft reibungsarm realisiert werden. Zudem kann der Expansionskörper dadurch mit einer besonders homogenen und schonend wirkenden Oberfläche und/oder Kontur ausgestattet werden. Der Runddraht kann hier ein einzelner Draht sein oder es können mehrere Drähte miteinander verflochten sein. In einer alternativen Realisierung der Abdeckvorrichtung kann das zumindest eine Geflecht aus ineinander verflochtenem Flachdraht gefertigt sein, wodurch eine Wandstärke des Expansionskörpers besonders dünn gestaltet werden kann. Generell wäre auch eine Kombination aus Runddraht und Flachdraht denkbar. Damit können die positiven Eigenschaften dieser beiden Ausgestaltungen vorteilhaft kombiniert werden.

Im montierten Zustand des zumindest einen Expansionskörpers in der Einführvorrichtung umfasst dieser ein proximales Ende, das im Benutzungszustand von einem distalen Ende der Einführvorrichtung entfernt ist, und ein distalen Ende, das im Benutzungszustand dem distalen Ende der Einführvorrichtung zugewandt ist. Hierbei stellt der Benutzerzustand den montierten bzw. den Zustand bei einer Anwendung bzw. Implantation des Implantats mit der Einführvorrichtung dar. In einer vorteilhaften Realisierung kann der zumindest eine Expansionskörper im Benutzerzustand in einer distalen Einführvorrichtung (siehe unten) am distalen Ende angeordnet sein, wodurch das Abdecken des freigegebenen Implantats konstruktiv einfach erfolgen kann.

Bevorzugt kann im Benutzerzustand in einer distalen Einführvorrichtung (siehe unten) am proximalen Ende des zumindest einen Expansionskörpers zumindest ein Griffsegment angeordnet sein. Das Griffsegment ist dazu ausgebildet, die Abdeckvorrichtung und den Expansionskörper unabhängig von den Einführelementen zu bewegen. Damit kann eine Bewegung des Expansionskörpers, insbesondere relativ zu einem der Einführelement bzw. dem äußeren Einführelement, zuverlässig auf diesen übertragen werden. Das Griffsegment ist damit an einem Teil jeder Abdeckvorrichtung angeordnet, der sich während der Implantation außerhalb des Körpers befindet. Das Griffsegment kann von einem Gehäuseanteil der Abdeckvorrichtung und/oder von einem separaten, an der Abdeckvorrichtung angeordneten oder angeformten Bauteil gebildet sein. Eine gute Bedienbarkeit und ein konstruktiv günstiger Aufbau sind gegeben, wenn das Griffsegment der Abdeckvorrichtung distal von zumindest einem Griffsegment eines der Einführelemente bzw. zumindest des äußeren Einführelements angeordnet ist.

Vorteilhafterweise umfasst der zumindest eine Expansionskörper im Benutzungszustand ein proximales Ende, das im Benutzungszustand von einem distalen Ende der Einführvorrichtung entfernt ist, und ein distalen Ende, das im Benutzungszustand dem distalen Ende der Einführvorrichtung zugewandt ist. Gemäß einer vorteilhaften Ausgestaltung kann zumindest das distale Ende des zumindest einen Expansionskörpers stumpf und/oder insbesondere mit geschlossenen Maschen ausgeführt sein. Hierdurch kann ein Anstoßen des distalen Endes des Expansionskörpers an z. B. Gefäßwände schonend und reizarm gestaltet werden. Geschlossenen Maschen sollen hier als verbundene Drähte verstanden werden.

Bevorzugt weist der zumindest eine Expansionskörper zumindest in einem Teilbereich eine Beschichtung auf. Dadurch kann der Expansionskörper speziell auf bestimmte Funktionen ausgelegt und/oder angepasst werden. Diese Beschichtung kann beispielsweise auf eine radiale Innenoberfläche, an der im abgedeckten Zustand zumindest ein Teil des Implantats radial anliegt, aufgebracht sein und/oder kann eine Gleitbewegung des Expansionskörpers relativ zum teilweise freigegebenen Implantat bei dem Abdecken unterstützen. Ein vielseitig einsetzbarer Expansionskörper kann bereitgestellt werden, wenn die Beschichtung von einem Kunststofffilm gebildet ist. Solch eine Beschichtung ist konstruktiv einfach auf den Teilbereich beispielsweise durch ein Sprüh- oder Tauchverfahren aufbringbar. Insbesondere ist dieser Kunststofffilm sehr dünn ausgeführt, wobei unter sehr dünn kleiner als 0,5 mm zu verstehen ist. Bevorzugt ist der Kunststofffilm zwischen 0,3 mm und 0,1 mm, insbesondere 0,2 mm, dick.

Vorteilhafterweise können das proximale Ende und das distale Ende der Abdeckvorrichtung aus unterschiedlichen Materialien gefertigt sein, wodurch Eigenschaften, wie Festigkeit, Elastizität, Biegeweiche, Gleitfähigkeit bspw. über eine Beschichtung, die eine Reibung erhöht oder vermindert, etc., individuell auf die Anforderungen des Abschnitts der Abdeckvorrichtung abgestimmt sein können. Das distale Ende stellt bei einem Einsatz der Abdeckvorrichtung in einer distalen Einführvorrichtung bevorzugt der Expansionskörper selbst dar.

Bei der Realisierung der Abdeckvorrichtung als ein Stabilisierungsschlauch der Einführvorrichtung kann eine sanfte und reibungsarme Zuführung des distalen Endes der Einführvorrichtung mit dem Implantat ermöglicht werden. Zudem erfüllt die Abdeckvorrichtung zwei Funktionen, nämlich die des Abdeckens bzw. die des Wiedereinhüllens des Implantats und die der stabilen Zuführung des distalen Endes der Einführvorrichtung, wodurch vorteilhaft Bauraum, Bauteile, Montageaufwand und Kosten eingespart werden können.

Gemäß eines weiteren Aspekts der Erfindung wird eine Einführvorrichtung zum Einführen eines medizinischen Implantats vorgeschlagen, welches durch eine Relativbewegung zwischen einem ersten und einem zweiten Einführelement freisetzbar ist, umfassend eine Abdeckvorrichtung zum Abdecken eines teilweise freigegebenen medizinischen Implantats, umfassend zumindest einen Expansionskörper, an dem eine erste radiale Erstreckung und zumindest eine zweite radiale Erstreckung einstellbar ist, wobei die zweite radiale Erstreckung des zumindest einen Expansionskörpers bei dem Abdecken des zumindest teilweise freigegebenen medizinischen Implantats an zumindest eine radiale Erstreckung des zumindest teilweise freigegebenen medizinischen Implantats anpassbar ist, wobei der zumindest eine Expansionskörpers zumindest in wenigstens einem axialen Abschnitt in Umfangsrichtung im Wesentlichen homogen aufweitbar ist.

Durch die erfindungsgemäße Ausgestaltung kann eine Einführvorrichtung bereitgestellt werden, mit der eine Abdeckung des zumindest teilweise freigegebenen Implantats schnell, komplikationsarm und schonend erfolgen kann. Dadurch kann auch eine Dauer eines Eingriffs an einem Patienten für diesen vorteilhaft verkürzt werden. Zudem kann anhand eines Resheathings eine Repositionierung des fehlplatzierten freigegebenen Implantats wegen der flexiblen Anpassung des Expansionskörpers an die Kontur des Implantats ermöglicht. Ferner kann ein Funktionstest des Implantats und im Defektfall eine Entfernung des missfunktionalen Implantats komfortabel unterstützt durch den Expansionskörper erfolgen. Des Weiteren kann eine Einführvorrichtung gestaltet werden, welche besonders kompakt ausgeführt ist. Damit weist die Einführvorrichtung gegenüber Systemen des Standes der Technik vorteilhaft einen geringeren Durchmesser auf. Zusätzlich kann durch eine solche Einführvorrichtung ein Risiko einer Deformation des Implantats sowie einer daraus resultierende Blockade der Einführvorrichtung eliminiert werden. Als ein weiterer Vorteil kann genannt werden, dass das Konzept des Expansionskörpers auf viele verschiedene Einführvorrichtungen und Implantate anwendbar ist.

Eine Einführvorrichtung kann beispielsweise in zwei Varianten ausgeführt sein. Diese Varianten unterscheiden sich darin, an welchem Ende die Freigabe des Implantats beginnt. Dies kann entweder an dem distalen Ende erfolgen, welches in Richtung des distalen Endes der Einführvorrichtung angeordnet ist oder an dem proximalen Ende, welches in Richtung des proximalen Endes des Einführvorrichtung angeordnet ist. Hierbei wird jede Einführvorrichtung in eine Einführrichtung bewegt. In diesem Zusammenhang soll unter der Einführrichtung die Richtung verstanden werden, entlang der die Einführvorrichtung mit der Abdeckvorrichtung und dem Implantat in den menschlichen und/oder tierischen Körper eingeführt wird. Insbesondere weist diese vom proximalen zum distalen Ende der Einführvorrichtung. Die Erfindung betrifft eine distale Einführvorrichtung.

Beginnt die Freigabe des Implantats an dessen distalen Ende ist die Abdeckvorrichtung einsetzbar für eine so genannte distale Einführvorrichtung. Hierbei ist das innere Einführelement (erstes Einführelement) mit einer Spitze der Einführvorrichtung, wie einer Katheterspitze, verbunden. Das äußere Einführelement (zweites Einführelement), welches radial um das innere Einführelement angeordnet ist, ist hingegen nicht mit der Katheterspitze verbunden und kann durch eine axiale Bewegung in Richtung des proximalen Endes der Einführvorrichtung relativ zu der Katheterspitze bewegt werden. Dadurch kann ein radial zwischen dem inneren und äußeren Einführelement angeordnetes Implantat zu dessen Implantation bzw. Expansion mit seinem distalen Ende zuerst freigelegt und/oder freigegeben werden.

Bevorzugt erstreckt sich der Expansionskörper in Umfangsrichtung um eines der Einführelemente. Zudem wird vorgeschlagen, dass der zumindest eine Expansionskörper der Abdeckvorrichtung radial um zumindest eines der Einführelemente und insbesondere um das äußere Einführelement gelegt ist. Durch diese Ausgestaltung kann der Expansionskörper vorteilhaft auf eine, insbesondere runde, Form des Einführelements angepasst sein, was eine raumsparende Verbindung zwischen dem Expansionskörper und dem Einführelement ermöglicht. Des Weiteren kann es vorteilhaft sein, wenn der zumindest eine Expansionskörper der Abdeckvorrichtung axial beweglich bezüglich zumindest einem der Einführelemente und insbesondere bevorzugt bezüglich des äußeren Einführelements angeordnet ist. Hierdurch kann das Abdecken des freigegebenen Implantats konstruktiv einfach und behinderungsfrei ermöglicht werden.

Bei einer Einführvorrichtung als distale Einführvorrichtung umfasst das zweite bzw. das äußere Einführelement eine Aufnahme für das Implantat. Hierbei liegt das Implantat an einer radialen Innenwand des zweiten Einführelements an, wodurch das zweite Einführelement als ein Klemmkörper für das Implantat fungiert. In diesem Zusammenhang soll unter einem "Klemmkörper" ein Körper verstanden werden, der mittels einer Klemmwirkung und/oder eines Kraftschlusses ein anderes Element, insbesondere das Implantat in der Einführvorrichtung in einer festgelegten Position hält. Das Implantat wird also in einem Klemmzustand verliersicher in der Einführvorrichtung gehalten. Im Klemmzustand hält beispielsweise eine Wechselwirkung zwischen einer Expansionskraft des Implantats und einer Klemmkraft des zweiten Einführelements des erstere in Position, wodurch ein Entgleiten des Implantats aus der Einführvorrichtung bzw. dem Klemmkörper verhindert wird. Zusätzlich kann der Klemmkörper eine von der Klemmwirkung separat ausgelegte Haltewirkung, beispielsweise durch eine Haftreibung aufweisen. Denkbar wäre hier auch eine Ausgestaltung des Klemmkörpers mit einer - zusätzlichen - speziellen Materialeigenschaft.

Eine räumlich komfortable Fixierung des Implantats kann bereitgestellt werden, wenn dieses in einer Schutzhülle des zweiten Einführelements fixiert ist. Diese Schutzhülle weist einen größeren radialen Durchmesser als das zweite Einführelement auf und stellt einen funktionalen Teil des zweiten Einführelements dar, d.h. sie kann zusammen mit diesem bewegt werden. Die Schutzhülle ist verliersicher mit dem zweiten Einführelement verbunden. Hierbei kann jede, dem Fachmann für sinnvoll erachtete Verbindungsart, wie ein Kraft-, ein Form- oder ein Stoffschluss, beispielsweise mittels Schweißen, Löten, Schrauben, Nageln oder Kleben, in Frage kommen. Bei einer Ausgestaltung von zwei verbundenen Bauteilen können Eigenschaften, wie Größe, Material, Beschichtung, Reibung etc., individuell auf die Anforderungen des Bauteils abgestimmt sein. Grundsätzlich kann die Schutzhülle auch einstückig mit dem zweiten Einführelement ausgeführt sein, wobei unter einstückig verstanden werden soll, dass die Schutzhülle und das zweite Einführelement von demselben Bauteil und/oder aus einem Guss gebildet sind und/oder nur unter Funktionsverlust von zumindest einem der Bauteile voneinander getrennt werden können. Die Schutzhülle kann zudem eine größere radiale Erstreckung aufweisen als ein Innendurchmesser des distalen Endes des zumindest einen Expansionskörpers in seiner ersten radialen Erstreckung. Dadurch kann die Einführvorrichtung von seinem proximalen zu seinem distalen Ende mit einer nahezu konstanten Erstreckung ausgeführt werden.

Gemäß einer vorteilhaften Realisierung der Erfindung ist das erste bzw. das innere Einführelement dazu ausgebildet, zumindest bei einer Expansion des Implantats dieses in Position zu halten. Dies kann mittels jedem, dem Fachmann für einsetzbar erachtetem Prinzip erfolgen, wie beispielweise einem Kraft- und/oder Formschluss, einer Haftreibung oder Ähnlichem. Bevorzugt kann ein distaler Endbereich des ersten Einführelements als ein Implantathalter ausgeführt sein, der zumindest eine Struktur, wie einen Haken, eine Ösen, einen Schlitze etc., aufweist, der dazu ausgebildet ist, mit einer Kontaktstruktur des Implantats zu interagieren. Diese sind beispielsweise als Ösen ausgeführt, die im montierten Zustand des Implantats in beispielsweise der distalen Einführvorrichtung an dem Ende des Implantats angeordnet sind, das zum proximalen Ende der Einführvorrichtung weist.

Gemäß einer vorteilhaften Ausgestaltung kann das Implantat als ein selbstexpandierendes Implantat ausgebildet sein, wodurch es bei Abwesenheit des äußeren Einführelements und/oder bei einem Austritt aus diesem selbsttätig öffnen kann. Durch das selbstexpandierende Implantat kann ein zusätzliches Expansionsmittel entfallen. Dadurch können vorteilhaft Raum und Montageaufwand für dieses eingespart werden. Hierdurch kann auch die Einführvorrichtung weniger komplex gestaltet werden. Grundsätzlich wäre es jedoch auch möglich ein ballonexpandierbares Implantat zu verwenden. Hierfür müsste die Einführvorrichtung jedoch entsprechend angepasst werden, was der Fachmann aufgrund seiner Fachkenntnis selbstständig löst.

Zudem wird ein Verfahren zum Abdecken eines zumindest teilweise freigegebenen Implantats mit einer Abdeckvorrichtung einer Einführvorrichtung vorgeschlagen. Das Verfahren weist zumindest die folgenden Schritte auf: Ausführen einer Relativbewegung zumindest eines Expansionskörpers der Abdeckvorrichtung bezüglich dem zumindest teilweise freigegebenen Implantats und dadurch Anpassen zumindest einer zweiten radialen Erstreckung des zumindest einen Expansionskörpers an zumindest eine radiale Erstreckung des zumindest teilweise freigegebenen medizinischen Implantats, wobei der zumindest eine Expansionskörpers zumindest in wenigstens einem axialen Abschnitt in Umfangsrichtung im Wesentlichen homogen aufgeweitet wird.

Durch die erfindungsgemäße Ausgestaltung kann ein Verfahren realisiert werden, mittels dem ein zumindest teilweise freigegebenes Implantat schnell, komplikationsarm und schonend platziert und letztendlich korrekt implantiert werden kann. Dadurch verkürzt sich auch eine Dauer eines Eingriffs an einem Patienten insbesondere für diesen vorteilhaft. Besonders vorteilhaft kann ein schon teilweise freigegebenes Implantat mittels des erfindungsgemäßen Verfahrens bedienerfreundlich in zumindest eines der Einführelemente zurückgezogen und repositioniert werden. Zudem kann an einem so freigegebenen Implantat ein Funktionstest durchgeführt werden und dieses kann im Defektfall entfernt werden. Des Weiteren kann bei einem solchen Verfahren eine Einführvorrichtung verwendet werden, die besonders kompakt ausgestaltet ist. Zusätzlich kann ein Risiko einer Deformation des Implantats sowie einer daraus resultierende Blockade der Einführvorrichtung eliminiert werden. Als ein weiterer Vorteil kann genannt werden, dass Verfahren auf viele verschiedene Einführvorrichtungen und Implantate anwendbar ist. Das erfindungsgemäße Verfahren stellt beispielsweise eine Transkatheter-Aortenklappenimplantations-Methode (TAVI-Methode) für eine Hybrid Bioprothese dar.

Zusätzlich weist das erfindungsgemäße Verfahren den Schritt: Rückexpandieren des zumindest teilweise freigegebenen medizinischen Implantats ausgelöst durch eine zu einer Innenachse der Einführvorrichtung wirkenden Rückstellkraft zumindest des Expansionskörpers der Abdeckvorrichtung, auf. Hierdurch kann das Rückexpandieren des Implantats konstruktiv einfach anhand einer im Expansionskörper vorhandenen Kraft bewerkstelligt werden, wodurch ein einfacher Mechanismus angewendet werden kann.

Die Erfindung ist nachfolgend beispielhaft, anhand von einem in Zeichnungen dargestellten Ausführungsbeispiel, näher erläutert. Es zeigen in schematischer Darstellung:
- Fig. 1: einen Schnitt durch ein bevorzugtes und günstiges Ausführungsbeispiel einer distalen Einführvorrichtung und einer erfindungsgemäßen Abdeckvorrichtung mit einem Expansionskörper und einem Implantat;
- Fig. 2: der Expansionskörper der Fig. 1 in einem Zustand mit einer ersten radialen Erstreckung;
- Fig. 3: der Expansionskörper der Fig. 1 mit einer eingestellten zweiten radialen Erstreckung an einem Ende;
- Fig.4: ein Ende des Expansionskörpers der Fig. 1 mit geschlossenen Maschen an einem Ende;
- Fig. 5: zwei sich kreuzende Anteile eines Runddrahts des Expansionskörpers der Fig. 1 in einer schematischen Darstellung;
- Fig. 6: eine Masche des Expansionskörpers der Fig. 1 in einer schematischen Darstellung;
- Fig. 7: die Masche des Expansionskörpers der Fig. 6 in einem unexpandierten (A) und in einem expandierten Zustand (B) in einer schematischen Darstellung;
- Fig. 8: der Expansionskörper der Fig. 1 in seinem unexpandierten geraden Zustand;
- Fig. 9: der Expansionskörper der Fig. 8 in einem gebogenen Zustand;
- Fig. 10: die Einführvorrichtung und der Abdeckkörper aus Fig. 1 mit vollständig platziertem Implantat vor einer Freigabe des Implantats;
- Fig. 11: die Einführvorrichtung und der Abdeckkörper aus Fig. 1 mit einem teilweise freigegebenen Implantat;
- Fig. 12: die Einführvorrichtung und der Abdeckkörper aus Fig. 1 mit dem teilweise freigegebenen Implantat abgedeckt durch den Expansionskörper der Fig. 3;
- Fig. 13: die Einführvorrichtung und der Abdeckkörper aus Fig. 1 mit erneut eingehülltem Implantat;
- Fig. 14: die Einführvorrichtung und der Abdeckkörper aus Fig. 1 mit vor einer Erneuten Freigabe des Implantats;
- Fig. 15: die Einführvorrichtung und der Abdeckkörper aus Fig. 1 mit dem teilweise freigegebenen Implantat abgedeckt durch den Expansionskörper der Fig. 3 bei einer Blockade während eines Einhüllprozesses;
- Fig. 16: eine Darstellung der Verfahrensschritte eines erfindungsgemäßen Einführund Abdeckverfahrens anhand eines Blockdiagramms;
- Fig. 17: die Einführvorrichtung und der Abdeckkörper aus Fig. 11 mit einem teilweise freigegebenen Implantat während einer Implantation an einem Implantationsort schematische Darstellung und;
- Fig. 18: die Einführvorrichtung und der Abdeckkörper aus Fig. 17 mit einem teilweise freigegebenen Implantat nach dem Abdecken mit dem Expansionskörper der Fig. 3 in einer schematischen Darstellung.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

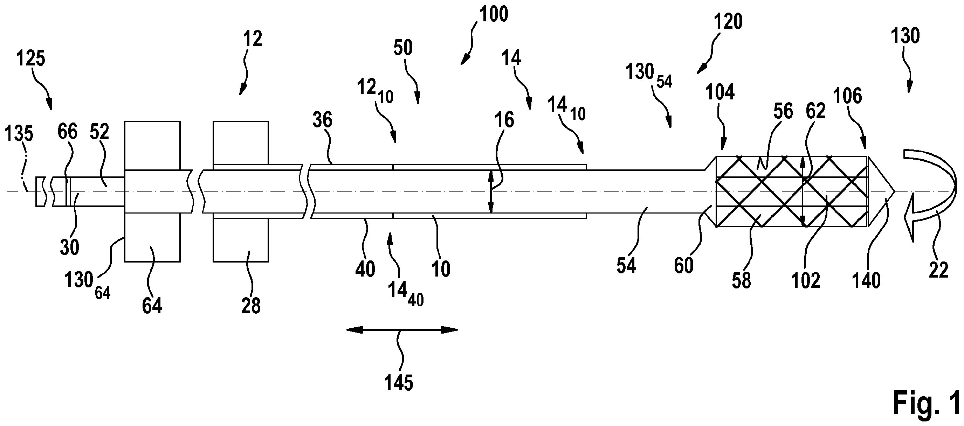

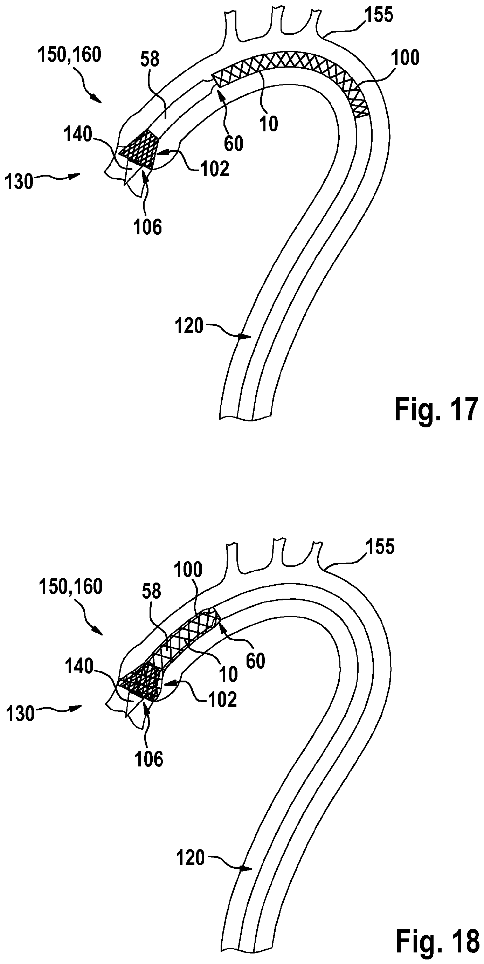

Fig. 1 zeigt einen Längsschnitt durch ein Ausführungsbeispiel einer Abdeckvorrichtung 100 einer distalen Einführvorrichtung 120 zum Einführen eines medizinischen selbstexpandierenden Implantats 102 in der Form eines Klappenimplantats bzw. eines Stents mit einer Klappenstruktur (nicht im Detail gezeigt). Die Einführvorrichtung 120 ist beispielsweise ein Katheter mit einem Schaftbereich 50 mit zwei koaxial angeordneten Einführelementen 52, 54, z.B. einem Innenschaft (Einführelement 52) und einem diesen umgebenden Außenschaft (Einführelement 54). Die Einführvorrichtung 120 ist im Anwenderbetrieb, also während einer Befestigung des Implantats 102 an der Einführvorrichtung 120 oder während der Implantation mit ihrem proximalen Ende 125 einem Anwender zugewandt. Das Implantat 102 ist an einem distalen Ende 130 des Schaftbereichs 50 zwischen Innenschaft und Außenschaft platziert und soll am Implantationsort 150, wie beispielsweise einem Aortenannulus, im tierischen oder menschlichen Körper freigegeben werden (vgl. Fig. 17 und 18).

Das Implantat 102 ist an dem vom Anwender abgewandten Ende 130 des Schaftbereichs 50 beispielsweise in der Nähe einer Katheterspitze 140 angeordnet. Das Implantat 102 ist beispielsweise um das innere Einführelement 52 gelegt bzw. mit nicht dargestellten Befestigungsstrukturen, wie bspw. Ösen, die an einem proximalen Ende 104 des Implantats 102 angeformt sind, an einem nicht im Detail gezeigten Implantathalter des inneren Einführelements 52 axial fixiert (nicht gezeigt). Dadurch ist das erste Einführelement 52 dazu ausgebildet, bei einer Expansion des Implantats 102 dieses axial in Position zu halten. Zu einer radialen Fixierung liegt das Implantat 102 an einer radialen Innenoberfläche 56 einer Schutzhülle 58 an. Diese Schutzhülle 58 ist über einen Verbindungsbereich 60 an einem distalen Ende 130₅₄ des äußeren Einführelements 54 angeordnet bzw. angeformt. Die Schutzhülle 58 weist eine größere radiale Erstreckung 62 auf wie das äußere Einführelement 54 und ist in seinen Dimensionen an das Implantat 102, insbesondere in dessen unexpandierten Zustand angepasst. Das Implantat 102 kann durch eine Relativbewegung zwischen dem ersten und dem zweiten Einführelement 52, 54 beginnend an einem distalen Ende 106 des Implantats 102 freigesetzt werden. Hierbei ist das innere Einführelement 52 mit der Katheterspitze 140 verbunden, das äußere Einführelement 54 hingegen nicht.

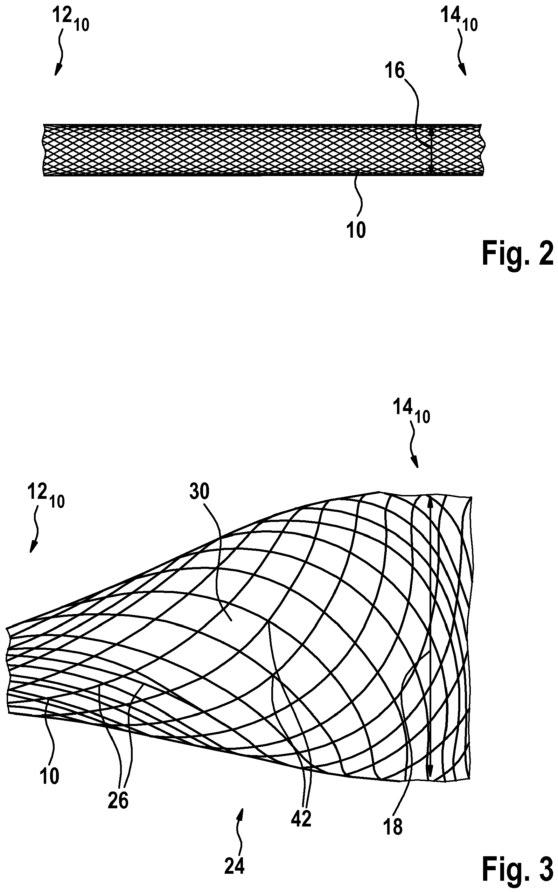

Die Abdeckvorrichtung 100 ist als ein Stabilisierungsschlauch 36 der Einführvorrichtung 120 ausgebildet und ist radial um das äußere Einführelement 54 gelegt. Zudem ist die Abdeckvorrichtung 100 axial beweglich bezüglich beider Einführelemente 52, 54 angeordnet. Die Abdeckvorrichtung 100 dient zum Abdecken des von der Einführvorrichtung 120 teilweise freigegebenen Implantats 102. Hierzu umfasst die Abdeckvorrichtung 100 einen Expansionskörper 10 an dem eine erste radiale Erstreckung 16 und eine zweite radiale Erstreckung 18 einstellbar ist (siehe unten und Fig. 2 und 3). Ferner weist die Abdeckvorrichtung 100 ein proximales Ende 12, das im Benutzungszustand von dem distalen Ende 130 der Einführvorrichtung 120 entfernt ist, und ein distalen Ende 14, das im Benutzungszustand dem distalen Ende 130 der Einführvorrichtung 120 zugewandt ist, auf. Der Expansionskörper 10 ist am distalen Ende 14 der Abdeckvorrichtung 100 angeordnet und im Benutzungszustand der Einführvorrichtung 120 proximal von der das Implantat 102 haltende Schutzhülle 58 platziert.

Am proximalen Ende 12 der Abdeckvorrichtung 10 ist ein Griffsegment 28 angeordnet. Dieses Griffsegment 28 ist distal von Griffsegmenten 64 der Einführelemente 52, 54 platziert, welche wiederum am proximalen Ende 125 der Einführvorrichtung 120 angeordnet sind. Hierbei ist lediglich das Griffsegment 64 des äußeren Einführelements 54 gezeigt. Alle Griffsegmente 28, 64 verbleiben während der Implantation außerhalb des Körpers.

In der Fig. 2 ist der Expansionskörper 10 im Zustand seiner ersten radialen Erstreckung 16 und in Fig. 3 im Zustand seiner zweiten radialen Erstreckung 18 gezeigt. Diese Erstreckung 16, 18 ist jeweils ein Durchmesser des Expansionskörpers 10 bzw. eines Bereichs davon. Die erste radiale Erstreckung 16 kann beispielsweise ca. 4,85 mm und die zweite radiale Erstreckung 18 z. B. 20,83 mm betragen. Somit ist die zweite radiale Erstreckung 18 etwa fünfmal größer wie die erste radiale Erstreckung 16 bzw. es ergibt sich eine Expansionsrate von ca. 430% (Ermittlung der Werte siehe unten).

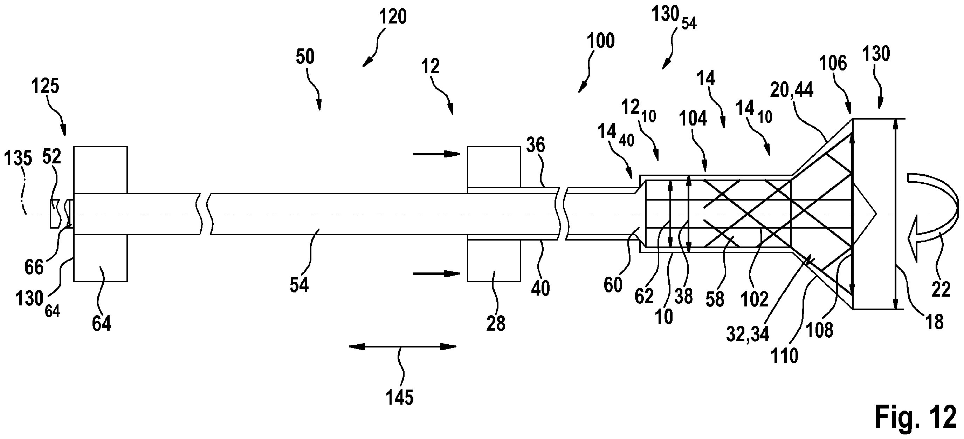

Hierbei ist die zweite radiale Erstreckung 18 lediglich an einem Enden 14₁₀ des Expansionskörpers 10 eingestellt, wobei dieses Ende 14₁₀ im Benutzungszustand in der Einführvorrichtung 120 dem distalen Ende 130 der Einführvorrichtung 120 zugewandt ist und somit sein distales Ende 14₁₀ darstellt. Das andere Ende 12₁₀ bzw. das Ende 12₁₀, welches im Benutzungszustand von einem distalen Ende 130 der Einführvorrichtung 120 entfernt ist, also das proximale Ende 12₁₀ darstellt, kann im Wesentlichen die erste radiale Erstreckung 16 beibehalten oder je nach Ausgestaltung des äußeren Einführelements 54, wie hier mit einer bezüglich des äußeren Einführelements 54 verbreiterten Schutzhülle 58, eine weitere radiale Erstreckung 38 annehmen (vgl. auch Fig. 12 und siehe unten). Im Bereich zwischen dem proximalen Ende 12₁₀ und dem distalen Ende 14₁₀ steigt die radiale Erstreckung im Wesentlichen gleichmäßig an. Dies ist jedoch auch abhängig von einer Außenkontur des äußeren Einführelements 54 und/oder der Schutzhülle 58. Das proximale Ende 14₁₀ des Expansionskörpers 10 ist mit einem Schaftmantel 40 des proximalen Teils 14 der Abdeckvorrichtung 100 verbunden. Hierbei kann jede, dem Fachmann für einsetzbar erachtete Verbindungsmethode, wie beispielsweise Schweißen, Löten, Schrauben, Nageln, Knoten oder Kleben, in Frage kommen (vgl. Fig. 1).

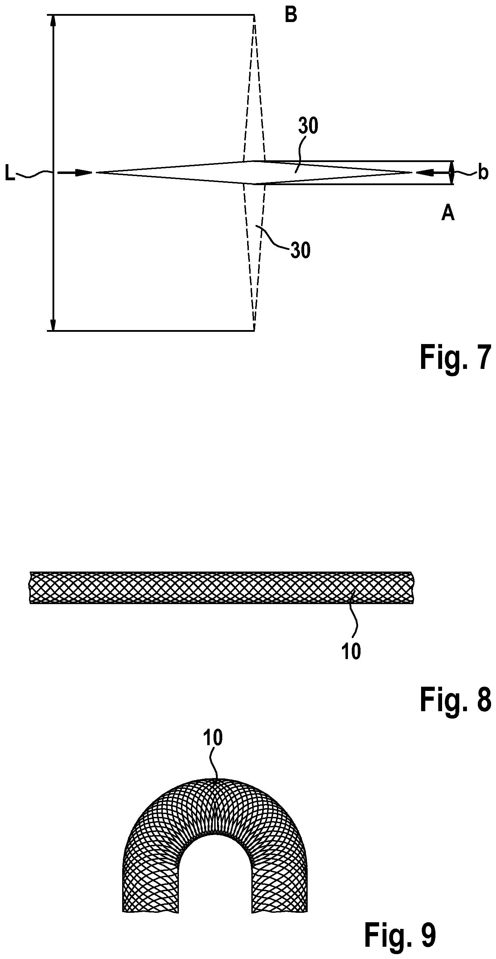

Wie insbesondere in Fig. 3 zu sehen ist, weist der Expansionskörper 10 ein Geflecht 24 bzw. ein Metallgeflecht auf, welches aus ineinander verflochtenem Runddraht 26 gefertigt ist. Dieser Runddraht 26 und damit der Expansionskörper 10 besteht beispielsweise aus Elgiloy. Zudem ist das Geflecht 24 reversibel faltbar ausgeführt, um die erste radiale Erstreckung 16 und die zweite radiale Erstreckung 18 einzustellen (vgl. auch Fig. 7).

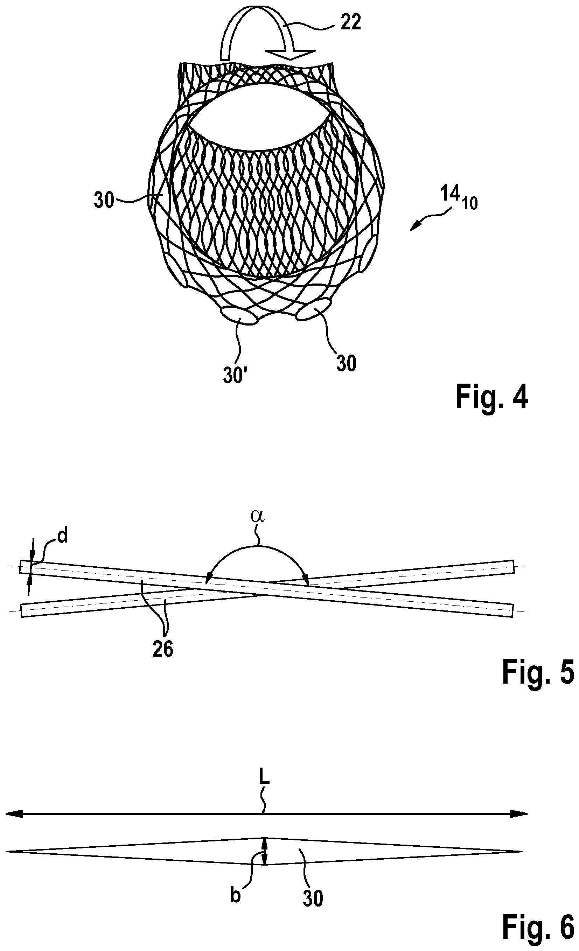

Fig. 4 zeigt eine Aufsicht auf das distale Ende 14₁₀ des Expansionskörpers 10. Um ein Verletzungsrisiko für einen Patienten während der Implantation und insbesondere während einem Verschieben der Abdeckvorrichtung 100 zu minimieren, ist das distale Ende 14₁₀ stumpf bzw. mit geschlossenen Maschen 30, 30' ausgeführt. Um die Abdeckvorrichtung 100 gut auf ihre Funktionen abzustimmen, sind das proximale Ende 12 und das distale Ende 14 aus unterschiedlichen Materialien gefertigt. Wie oben beschrieben, besteht der am distalen Ende 14 angeordnete Expansionskörper 10 aus Elgiloy. Das proximale Ende 12 kann beispielsweise aus einem stabilen aber biegsamen Kunststoff gefertigt sein. Das Griffsegment 28 hingegen ist bevorzugt aus einem harten, formstabilen Werksstoff gefertigt.

In den Fig. 5 bis 7 sind die Dimensionen des Expansionskörpers 10 bzw. seiner Bestandteile veranschaulicht. Ein Durchmesser d des Runddrahts 26 beträgt beispielsweise 0,2 mm und ein Kreuzungswinkel α, der im unexpandierten Zustand des Expansionskörpers zwischen den Runddrähten 26 eingestellt ist, z. B. 175° (siehe Fig. 5). Eine axiale Länge L einer zwischen den Enden 12₁₀ und 14₁₀ des Expansionskörpers 10 liegende Masche 30 beträgt bspw. 5 mm (siehe Fig. 3 und 6). Eine Breite b der Masche 30 in Umfangsrichtung 22 des Expansionskörpers 10 ist beispielsweise 0,9 mm (siehe Fig. 3, 4 und 6). Weist der Expansionskörper 10 bzw. das Geflecht 24 in Umfangsrichtung 22 nun z. B. 15 Maschen 30 bzw. Kreuzungspunkte 42 auf, ergibt sich im unexpandierten Zustand ein Umfang des Expansionskörpers 10 von 13,5 mm (15 x die Materialstärke d des Runddrahts 24 von 0,2 mm + 15 x die Breite b von 0,9 mm, vgl. Fig. 3, 5 und 7A) und im expandierten Zustand ein Umfang des Expansionskörpers 10 von 78 mm (15 x die Materialstärke d des Runddrahts 24 von 0,2 mm + 15 x die Länge L von 5 mm, vgl. Fig. 3, 5 und 7B). Somit kann der Umfang des Expansionskörpers 10 im expandierten Zustand einen etwa fünfmal größeren Wert einnehmen wie der Umfang des Expansionskörpers 10 im unexpandierten Zustand bzw. es ergibt sich eine Expansionsrate von ca. 470% (Die Diskrepanz zu der oben angegebenen Expansionsrate ergibt sich dadurch, dass oben die Innendurchmesser verglichen wurden und hier die Außendurchmesser relevant sind.).

In Fig. 8 ist ein Abschnitt des Expansionskörpers 10 in seinem unexpandierten geraden Zustand gezeigt, wie er insbesondere in einem unmontierten Zustand auf der Einführvorrichtung 120 vorliegt. Fig. 9 zeigt diesen Bereich des Expansionskörpers 10 in einem gebogenen Zustand, wie er insbesondere bei dem Zuführen über beispielsweise einem Aortenbogen 155 der Einführvorrichtung 120 zum Implantationsort 150 vorliegen kann (vgl. auch Fig. 17).

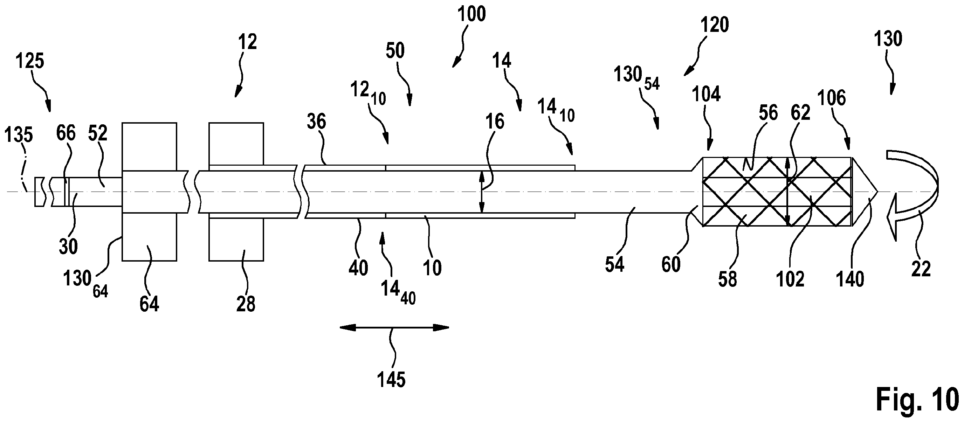

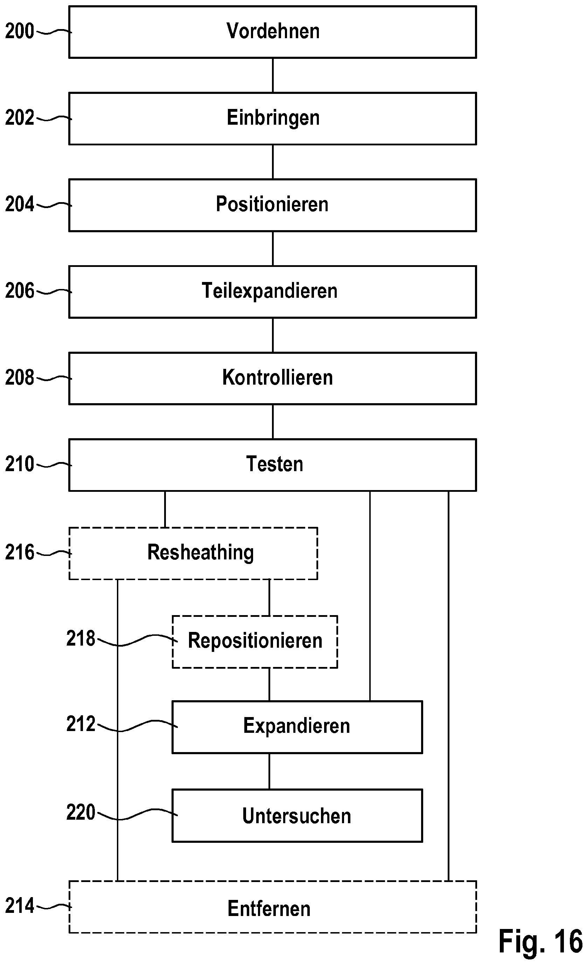

Im Folgenden ist das Einführen des medizinischen Implantats 102 mit Hilfe der Einführvorrichtung 120 exemplarisch für die Implantation einer künstlichen Herzklappe an einem Aortenannulus und ein Verfahren zum Abdecken des teilweise freigelegten Implantats 102 mit der Abdeckvorrichtung 10 anhand der Fig. 10 bis 15 sowie Fig. 17 und 18 und des Blockdiagramms der Fig. 16 beschrieben. Das Implantat 102 ist, wie oben beschrieben und in Fig. 10 gezeigt ist, in/an der Einführvorrichtung 120 montiert.

Vor dem Einführen der Einführvorrichtung 120 wird die native Aortenklappe 160 in Schritt 200 Vordehnen mit einem so genannten Valvuloplastie-Ballon vorgedehnt. Anschließend wird die so vorbereitete Einführvorrichtung 120 in Schritt 202 Einbringen zu der Implantation des Implantats 102 im Körper in einer bekannten Weise mit Hilfe eines nicht näher gezeigten Führungselements, eines so genannten guide wires, in den Körper eingeführt und in Schritt 204 Positionieren positioniert. Hierbei sollte eine Klappenebene der nicht gezeigten Klappenstruktur bündig mit dem Aortenannulus der natürlichen Klappe 160 angeordnet sein (vgl. Fig. 16 und 17, natürliche Klappe nicht im Detail gezeigt).

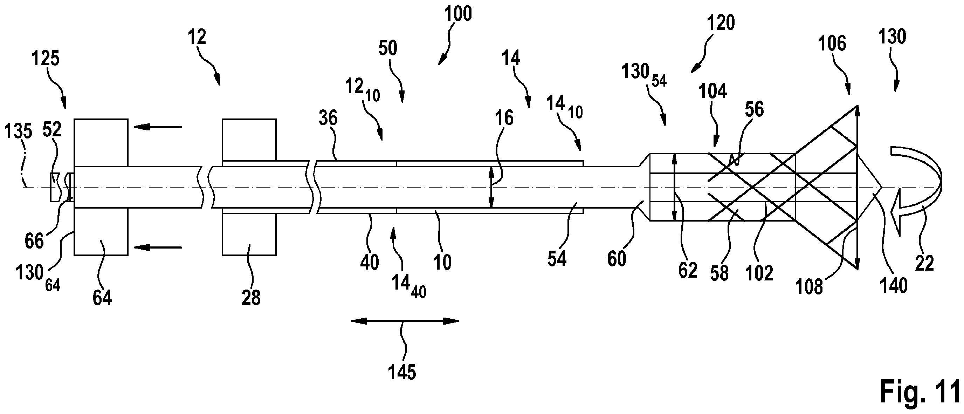

Nun beginnt ein Platzieren des Implantats 102. Dabei wird in einem ersten Schritt 206 Teilexpandieren das distale Ende 106 Implantats 102 freigelegt, wodurch dieser selbsttätig expandiert (vgl. Fig. 11 und 17). Hierbei wird das zweite Einführelement 54 mit dem Griffsegment 64 in Richtung des proximalen Endes 125 der Einführvorrichtung 120 gezogen (siehe Pfeile in Fig. 11), wodurch das zweite Einführelement 54 eine Relativbewegung in axialer Richtung 145 bezüglich des ersten Einführelements 52 und der Abdeckvorrichtung 100 vollzieht. Eventuell müssen hierfür das erste Einführelement 52 und die Abdeckvorrichtung 100 an ihren Griffsegmenten 28, 64 axial fixiert werden. Die Ziehbewegung erfolgt hierbei so lange, bis sich ein proximales Ende 130₆₄ des Griffsegments 64 des zweiten Einführelements 54 auf einer axialen Höhe eines Stopps 66 des ersten Einführelements 52 befindet (siehe die axialen Höhen des Griffsegments 28 der Fig. 10 und 11). Somit wird die Relativbewegung anhand des Stopps 66 limitiert (siehe Fig. 11). Dieser Stopp 66 stellt beispielsweise eine Markierung dar.

Damit ist das Implantat 102 teilweise freigelegt und das distale Ende 106 hat in seinem so expandierten Zustand eine radiale Erstreckung 108 angenommen. Hierbei ist die radiale Erstreckung 108 minimal kleiner wie die zweite radiale Erstreckung 18 des Expansionskörpers 10, die dieser beim Abdecken des teilweise freigelegten Implantats 102 annehmen wird (siehe unten und Fig. 12).

An dieser Stelle kann in Schritt 208 Kontrollieren eine Positionskontrolle des expandierten distalen Endes 106 des Implantats 102 durchgeführt werden. Zusätzlich können nun in Schritt 210 Testen Funktionstests des Implantats 102 oder dessen Klappenstruktur ausgeführt werden. Befindet sich das distale Ende 106 des Implantats 102 in der erwünschten und korrekten Position, wie dies in Fig. 16 durch ein Häkchen symbolisiert ist, wird in einem Schritt 212 Expandieren das durch weiteres Zurückziehen des zweiten Einführelements 54 in Richtung des proximalen Endes 125 der Einführvorrichtung 120 das proximale Ende 104 des Implantats 102 freigelegt (nicht im Detail gezeigt, siehe Fig. 16). Bei einer festgestellten Missfunktion kann das Implantat 102 in Schritt Entfernen 214, wie dies in Fig. 16 durch ein durchkreuztes Häkchen symbolisiert ist, wieder aus dem Körper entfernt werden (siehe Fig. 16). Da Schritt 214 einen alternativen Verfahrensschritt darstellt, ist er in Fig. 16 als Kasten mit gestrichelter Umrandung gezeigt.

Wird eine Fehlposition des distalen Endes 106 festgestellt oder funktioniert die Klappenstruktur in der Position ungenügend, kann dies in den nächsten Schritten behoben werden, wie dies in Fig. 16 durch ein durchkreuztes Häkchen symbolisiert ist. Hierbei muss erst in einem Schritt 216 Resheathing eine Wiedereinhüllen oder ein so genanntes Resheathing des bereits expandierten Endes 106 des Implantats 102 durchgeführt werden. Hierfür muss das freigegebene Ende 106 des Implantats 102 erst wieder in seinen unexpandierten Zustand überführt werden. Dabei wird der Expansionskörper 10 zum Abdecken des teilweise freigegebenen Implantats 102 durch Ausführen einer Relativbewegung des Expansionskörpers 10 bezüglich des zumindest teilweise freigegebenen Implantats 102 sowie bezüglich des zweiten Einführelements 54 in axialer Richtung 145 über das bereits expandierte distale Ende 106 des Implantats 102 geschoben, wie dies in Fig. 12 und 18 gezeigt ist (siehe Pfeile in Fig. 12).

Dadurch kommt es zu einem Anpassen des Expansionskörpers 10 zumindest in seiner zweiten radialen Erstreckung 18 an die radiale Erstreckung 108 des teilweise freigegebenen Implantats 102 bzw. ein axialer Abschnitt 20 bzw. in diesem Ausführungsbeispiel ein distales Drittel 44 des Expansionskörpers 10 an dessen distalem Ende 14₁₀ passt sich an eine Außenkontur 110 des freigegebenen distalen Endes 106 des Implantats 102 an. Hierbei wird der Expansionskörpers 10 in einem axialen Abschnitt 20 bzw. in seinem distalen Drittel 44 in Umfangsrichtung 22 und zusätzlich in axialer Richtung 145 homogen aufgeweitet. Der Expansionskörper 10 weitet sich sozusagen wie ein Trichter um das teilweise freigegebene Implantat 102 auf. Dies ist einfach realisierbar, da sich gezeigt hat, dass eine Reibung zwischen einem Material des Implantats 102, wie beispielsweise Nitinol, und dem Expansionskörper 10 geringer ist, als eine Reibung zwischen dem Implantat 102 (Nitinol) und einer Aortenwand und/oder einer Stenose am Implantationsort 150. Zur Unterstützung der Relativbewegung zwischen dem Expansionskörper 10 und dem Implantat 102 weist ein Teilbereich 32 und zwar eine Innenoberfläche im distalen Drittel 44 des Expansionskörpers 10 eine Beschichtung 34 auf, die von einem dünnen Kunststofffilm gebildet ist. Dieser Kunststofffilm besteht aus Teflon und weist eine Dicke von 0,2 mm auf.

Die Bewegung der Abdeckvorrichtung 100 wird durch ein Anschlagen des distalen Endes 14₄₀ des Schaftmantels 40 an den konisch nach außen verlaufenden Verbindungsbereich 60 zwischen dem zweiten Einführelement 54 und der Schutzhülle 58 limitiert. Hierbei sind die Dimensionen, insbesondere die Längen, der Bauteile (Schaftmantel 40, Expansionskörper 10, Implantat 102, Schutzhülle 58, Position des Stopps 66,) bzw. ist deren Bewegungsspielraum so aufeinander abgestimmt, dass das distale Ende 14₁₀ des Expansionskörpers 10 im Wesentlichen auf derselben axialen Höhe positioniert wird, wie das distale Endes 106 des Implantats 102 nach dem Teilexpandieren. Zudem expandiert bei dem Bewegen der Abdeckvorrichtung 100 auch das proximale Ende 12₁₀ des Expansionskörpers 10. Dieser nimmt die weitere radiale Erstreckung 38 an, die sich an die radial verbreiterte Schutzhülle 58 anpasst.

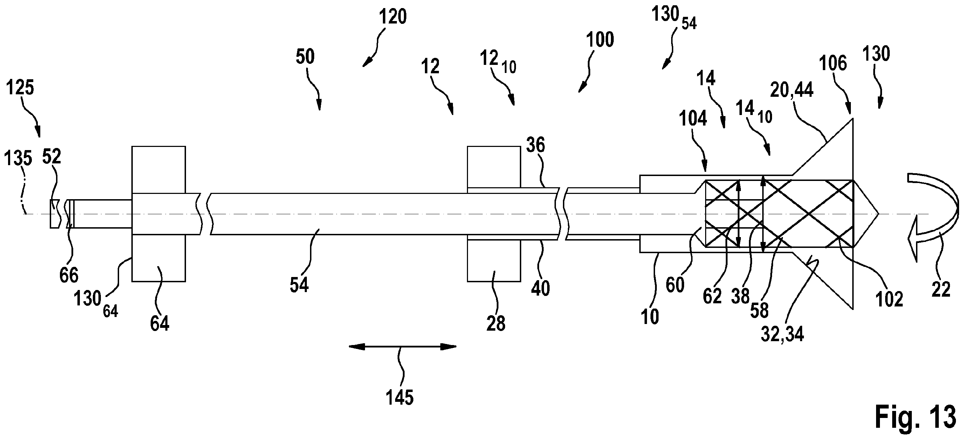

Da der Expansionskörper 10 bei eingestellter zweiter radialer Erstreckung 18 eine zu einer Innenachse 135 der Einführvorrichtung 120 wirkenden Rückstellkraft aufweist, die dazu ausgelegt ist, ein Rückexpandieren des teilweise freigegebenen Implantats 102 auszulösen, erfolgt nun das Rückexpandieren des teilweise freigegebenen Implantats 102, bei dem das expandierte distale Ende 106 des Implantats 102 radial nach innen gedrückt wird und dieses in seinen unexpandierten Zustand zurück kehrt (siehe Fig. 13).

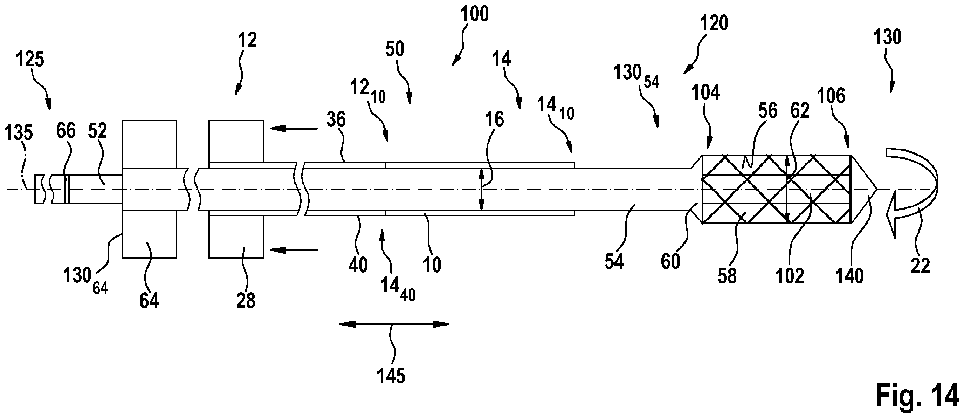

Nun kann das eigentliche Resheathing 216 erfolgen. Dabei wird das erste Einführelement 52 in axialer Richtung 145 in Richtung des proximalen Endes 125 der Einführvorrichtung 120 gezogen (siehe Pfeile in Fig. 13). Dadurch tritt das zuvor durch die Rückstellkraft des Expansionskörpers 10 zusammengedrückte distale Endes 106 des Implantats 102 wieder in die Schutzhülle 58 des zweiten Einführelements 54 ein. Das distale Drittel 44 des Expansionskörpers 10 ist in Fig. 13 zur besseren Darstellbarkeit des zusammengedrückten und sich wieder in der Schutzhülle 58 befindlichen Implantats 102 expandiert gezeigt, obwohl das distale Drittel 44 hier die weitere radiale Erstreckung 38, angepasst auf die Erstreckung 62 der Schutzhülle 58, angenommen hat. Nachfolgend wird die Abdeckvorrichtung 100 in axialer Richtung 145 in Richtung des proximalen Endes 125 der Einführvorrichtung 120 gezogen (siehe Pfeile in Fig. 14), wodurch der Expansionskörper 10 wieder die erste radiale Erstreckung 16 annimmt und sich die Abdeckvorrichtung 100 so wieder in ihrer Ausgangsposition befindet (vgl. Fig. 14).

Nun kann das distale Ende 106 des Implantats 102 in Schritt 218 Repositionieren durch erneutes Zurückziehen des zweiten Einführelements 54 nochmals positioniert werden, wie dies in Fig. 16 durch ein Häkchen symbolisiert ist (vgl. Fig. 11). Da die Schritte 216 und 218 alternative Verfahrensschritte darstellen, sind sie in Fig. 16 als Kasten mit gestrichelter Umrandung gezeigt. Befindet sich das distale Ende 106 des Implantats 102 nun in der erwünschten und korrekten Position kann es endgültig freigegeben werden (symbolisiert dies in Fig. 16 durch ein Häkchen). Hierfür wird das Implantat 102 vom Implantathalter gelöst. Dies erfolgt beispielsweise durch radiales Zurückziehen von Haken des Implantathalters aus den Befestigungsösen des proximalen Endes 104 des Implantats 102 (nicht gezeigt). Im Anschluss kann in einem Schritt 212 Expandieren das vollständige Implantat 102 freigesetzt werden (nicht im Detail gezeigt, vgl. Fig. 16). Das Expandieren des proximalen Endes 104 des Implantats 102 wird hierbei durch weiteres Zurückziehen des zweiten Einführelements 54 in Richtung des proximalen Endes 125 der Einführvorrichtung 120 ausgelöst, wodurch sich dieses selbsttätig expandiert. Letztlich wird in Schritt 214 Entfernen die Einführvorrichtung 120 zurückgezogen und aus dem Körper entfernt (vgl. Fig. 16). Das Implantat 102 verbleibt vollständig positioniert im Körper (nicht gezeigt). Um die erfolgreiche Implantation zu verifizieren kann dann in Schritt 220 Untersuchen ein Aortogramm aufgenommen werden.

Alternativ könnte bei dem eigentlichen Resheathing 216 auch das zweite Einführelement 54 in axialer Richtung 145 in Richtung des distalen Endes 130 der Einführvorrichtung 130 geschoben werden, wodurch das Implantat 102 wieder komplett in der Schutzhülle 58 angeordnet wird. Die Ziehbewegung erfolgt hierbei so lange bis das Implantat 102 vollständig freigesetzt ist.

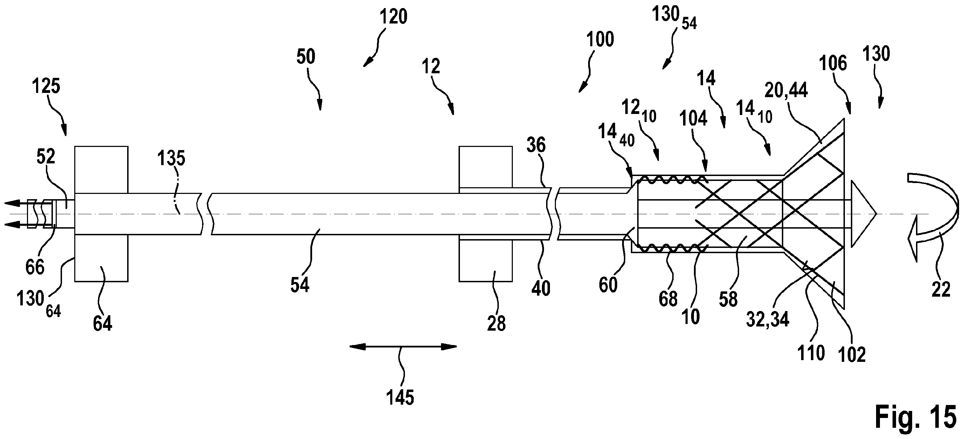

Es wäre auch möglich, dass in dem Schritt 216 Resheathing das Implantat 102 durch eine Bewegung des ersten Einführelements 52 in axialer Richtung 145 in Richtung des proximalen Endes 125 der Einführvorrichtung 120 nicht korrekt in die Schutzhülle 58 zurück bewegt werden kann (siehe Pfeile in Fig. 15). Alternativ könnte sich auch bei einer Bewegung des zweiten Einführelements 54 in axialer Richtung 145 in Richtung des distalen Endes 130 der Einführvorrichtung 120 das Implantat 102 verklemmen, wodurch es nicht abgedeckt werden könnte. In beiden Fällen kommt es dadurch in einem Bereich 68 der Schutzhülle 58 zu einer Faltung wodurch das Resheathing unmöglich ist, wie dies in Fig. 16 durch ein durchkreuztes Häkchen symbolisiert ist. Hierbei kann der Expansionskörper 10 nun helfen, in Schritt 214 Entfernen bei einem Zurückziehen der Einführvorrichtung 120 mit dem Implantat 102 eine Hülle um das Implantat 102 zu bilden, die verhindert, dass es bei der Explantation zu Verletzungen des Patienten kommt.

Prinzipiell kann eines der Bauteile/Bestandteil der Einführvorrichtung 120 oder des Implantats 102 auch aus einem röntgensichtbaren Metall, wie beispielsweise Edelstahl, Tantal, Gold oder Platin gebildet sein. Somit könnte anhand eines hier nicht gezeigten Röntgengeräts während der Implantation des Implantats 102 mittels der Einführvorrichtung 120 ein Fortschritt beispielsweise der Katheterspitze 140 oder eines der Einführelemente 52,54 und damit des Implantats 102 sowie eine korrekte Position des Implantats 102 an dem Implantationsort 150 überwacht werden.

### Bezugszeichen

- 10: Expansionskörper
- 12: Ende
- 14: Ende
- 16: Erstreckung
- 18: Erstreckung
- 20: Abschnitt
- 22: Umfangsrichtung
- 24: Geflecht
- 26: Runddraht
- 28: Griffsegment
- 30: Masche
- 32: Teilbereich
- 34: Beschichtung
- 36: Stabilisierungsschlauch
- 38: Erstreckung
- 40: Schaftmantel
- 42: Kreuzungspunkt
- 44: Drittel
- 50: Schaftbereich
- 52: Einführelement
- 54: Einführelement
- 56: Innenoberfläche
- 58: Schutzhülle
- 60: Verbindungsbereich
- 62: Erstreckung
- 64: Griffsegment
- 66: Stopp
- 68: Bereich
- 100: Abdeckvorrichtung
- 102: Implantat
- 104: Ende
- 106: Ende
- 108: Erstreckung
- 110: Außenkontur
- 120: Einführvorrichtung
- 125: Ende
- 130: Ende
- 135: Innenachse
- 140: Katheterspitze
- 145: Richtung
- 150: Implantationsort
- 155: Aortenbogen
- 160: Aortenklappe
- 200: Vordehnen
- 202: Einbringen
- 204: Positionieren
- 206: Teilexpandieren
- 208: Kontrollieren
- 210: Testen
- 212: Expandieren
- 214: Entfernen
- 216: Resheathing
- 218: Repositionieren
- 220: Untersuchen
- b: Breite
- d: Durchmesser
- L: Länge
- α: Kreuzungswinkel

## Patentansprüche

1. Einführvorrichtung (120) zum Einführen eines medizinischen Implantats (102), welches durch eine Relativbewegung zwischen einem ersten und einem zweiten Einführelement (52; 54) freisetzbar ist, umfassend eine Abdeckvorrichtung (100) zum Abdecken eines teilweise freigegebenen medizinischen Implantats (102) umfassend zumindest einen Expansionskörper (10), an dem eine erste radiale Erstreckung (16) und zumindest eine zweite radiale Erstreckung (18) einstellbar ist, wobei die zweite radiale Erstreckung (18) des zumindest einen Expansionskörpers (10) bei dem Abdecken des zumindest teilweise freigegebenen medizinischen Implantats (102) an zumindest eine radiale Erstreckung (108) des zumindest teilweise freigegebenen medizinischen Implantats (102) anpassbar ist, wobei der zumindest eine Expansionskörpers (10) zumindest in wenigstens einem axialen Abschnitt (20) in Umfangsrichtung (22) im Wesentlichen homogen aufweitbar ist, wobei der zumindest eine Expansionskörper (10) der Abdeckvorrichtung (100) radial um zumindest eines der Einführelemente (52,54) gelegt ist und axial beweglich bezüglich zumindest einem der Einführelemente (52, 54) angeordnet ist,
wobei die Einführvorrichtung (10) derart ausgestaltet ist, dass die Freigabe des Implantats (102) an dessen distalen Ende (106) beginnt,
wobei das erste Einführelement (52) als inneres Einführelement mit einer Spitze (140) der Einführvorrichtung (10) verbunden ist,
wobei das zweite Einführelement (54) als äußeres Einführelement radial um das erste, innere Einführelement (52) angeordnet und nicht mit der Katheterspitze (140) verbunden ist und durch eine axiale Bewegung in Richtung des proximalen Endes (125) der Einführvorrichtung (10) relativ zur Katheterspitze (140) beweglich ist, so dass ein radial zwischen dem inneren und äußeren Einführelement angeordnetes Implantat (102) zur Implantation mit seinem distalen Ende (106) zuerst freigegeben werden kann,
wobei das zweite, äußere Einführelement (54) eine Aufnahme für das Implantat umfasst, wobei das Implantat an einer radialen Innenwand (56) des zweiten Einführelementes (54) anliegt, so dass das zweite Einführelement (54) als Klemmkörper für das Implantat (102) fungiert, **dadurch gekennzeichnet, dass**
die Abdeckvorrichtung als ein Stabilisierungsschlauch (36) der Einführvorrichtung (120) ausgebildet ist und
radial um das zweite, äußere Einführelement gelegt ist.

2. Einführvorrichtung nach Anspruch 1, wobei das Implantat (102) als ein selbstexpandierendes Implantat (102) ausgebildet ist.

3. Einführvorrichtung nach Anspruch 1 oder 2, wobei der zumindest eine Expansionskörper (10) zumindest bei eingestellter zweiter radialer Erstreckung (18) eine zu einer Innenachse (135) der Einführvorrichtung (120) wirkenden Rückstellkraft aufweist, die dazu ausgelegt ist, ein Rückexpandieren des zumindest teilweise freigegebenen medizinischen Implantats (102) auszulösen.

4. Einführvorrichtung nach einem der vorhergehenden Ansprüche, wobei die zumindest zweite radiale Erstreckung (18) des zumindest einen Expansionskörpers (10) bezüglich der ersten radialen Erstreckung (16) des zumindest einen Expansionskörpers (10) bevorzugt zweimal, vorteilhaft dreimal und besonders bevorzugt mehr als fünfmal größer ist.

5. Einführvorrichtung nach einem der vorhergehenden Ansprüche, wobei der zumindest eine Expansionskörper (10) zumindest ein Geflecht (24) aufweist, welches reversibel faltbar ausgeführt ist, um die erste radiale Erstreckung (12) und die zumindest zweite radiale Erstreckung (18) einzustellen.

6. Einführvorrichtung nach einem der vorhergehenden Ansprüche, wobei der zumindest eine Expansionskörper (10) zumindest ein Geflecht (24) aufweist, welches aus ineinander verflochtenem Runddraht (26) gefertigt ist.

7. Einführvorrichtung nach einem der vorhergehenden Ansprüche, wobei der zumindest eine Expansionskörper (10) aus Elgiloy gefertigt ist.

8. Einführvorrichtung nach einem der vorhergehenden Ansprüche, mit einer Abdeckvorrichtung umfassend ein proximales Ende (12), das im Benutzungszustand von einem distalen Ende (130) der Einführvorrichtung (120) entfernt ist, und ein distalen Ende (14), das im Benutzungszustand dem distalen Ende (130) der Einführvorrichtung (120) zugewandt ist, wobei der zumindest eine Expansionskörper (10) am distalen Ende (14) angeordnet ist und/oder am proximalen Ende (12) zumindest ein Griffsegment (28) angeordnet ist.

9. Einführvorrichtung nach einem der vorhergehenden Ansprüche, wobei der zumindest eine Expansionskörper (10) im Benutzungszustand ein proximales Ende (12₁₀), das im Benutzungszustand von einem distalen Ende (130) der Einführvorrichtung (120) entfernt ist, und ein distalen Ende (14₁₀), das im Benutzungszustand dem distalen Ende (130) der Einführvorrichtung (120) zugewandt ist, aufweist, und wobei zumindest das distale Ende (14₁₀) des zumindest einen Expansionskörpers (10) stumpf und/oder insbesondere mit geschlossenen Maschen (30, 30') ausgeführt ist.

10. Einführvorrichtung nach einem der vorhergehenden Ansprüche, wobei der zumindest eine Expansionskörper (10) zumindest in einem Teilbereich (32) eine Beschichtung (34) aufweist und/oder wobei die Beschichtung (34) von einem Kunststofffilm gebildet ist.

11. Einführvorrichtung nach einem der vorhergehenden Ansprüche, umfassend ein proximales Ende (12), das im Benutzungszustand von einem distalen Ende (130) der Einführvorrichtung (120) entfernt ist, und ein distalen Ende (14), das im Benutzungszustand dem distalen Ende (130) der Einführvorrichtung (120) zugewandt ist, wobei das proximale Ende (12) und das distale Ende (14) aus unterschiedlichen Materialien gefertigt sind.

## Claims

1. An introduction device (120) to introduce a medical implant (102) and allow its release by a relative motion between a first and a second introduction element (52; 54), this introduction device comprising a covering device (100) to cover a partly released medical implant (102), this covering device comprising at least one expansion body (10), which can be set to a first radial dimension (16) and at least one second radial dimension (18), the second radial dimension (18) of the at least one expansion body (10) being adaptable to at least one radial dimension (108) of the at least partly released medical implant (102) as it is being covered, the at least one expansion body (10) being essentially homogeneously widenable in the peripheral direction (22) at least in at least one axial section (20), the at least one expansion body (10) of the covering device (100) being laid radially around at least one of the introduction elements (52,54) and axially movable with respect to at least one of the introduction elements (52, 54),
the introduction device (10) being designed so that the release of the implant (102) begins at its distal end (106),
the first introduction element (52) in the form of the inner introduction element being connected with a tip (140) of the introduction device (10),
the second introduction element (54) in the form of the outer introduction element being arranged radially around the first, inner introduction element (52) and not being connected with the catheter tip (140) and being movable relative to the catheter tip (140) by an axial movement toward the proximal end (125) of the introduction device (10), so that an implant (102) that is arranged radially between the inner and outer introduction elements for its implantation can first be released with its distal end (106),
the second, outer introduction element (54) comprising a receptacle for the implant, the implant lying against a radial inner wall (56) of the second introduction element (54), so that the second introduction element (54) functions as a clamping body for the implant (102), **characterized in that**
the covering device is in the form of a stabilization tube (36) of the introduction device (120), and
is laid radially around the second, outer introduction element.

2. An introduction device according to claim 1, wherein the implant (102) is in the form of a self-expanding implant (102).

3. An introduction device according to claim 1 or 2, wherein the at least one expansion body (10) has, at least if it is set to the second radial dimension (18) a restoring force acting toward an internal axis (135) of the introduction device (120), this restoring force being designed to recompress the at least partly released medical implant (102).

4. An introduction device according to any one of the preceding claims, wherein the at least second radial dimension (18) of the at least one expansion body (10) is preferably two times, advantageously three times, and especially preferably more than five times bigger than the first radial dimension (16) of the at least one expansion body (10).

5. An introduction device according to any one of the preceding claims, wherein the at least one expansion body (10) has at least one braid (24) that is reversibly foldable, to allow it to be set to the first radial dimension (12) and the at least second radial dimension (18).

6. An introduction device according to any one of the preceding claims, wherein the at least one expansion body (10) has at least one braid (24), which is made of round wire (26) that is braided together.

7. An introduction device according to any one of the preceding claims, wherein the at least one expansion body (10) is made of Elgiloy.

8. An introduction device according to any one of the preceding claims, with a covering device comprising a proximal end (12), which is, in the state in which it is used, remote from a distal end (130) of the introduction device (120), and a distal end (14), which is, in the state in which it is used, facing the distal end (130) of the introduction device (120), the at least one expansion body (10) being arranged at the distal end (14) and/or at least one handle segment (28) being arranged at the proximal end (12).

9. An introduction device according to any one of the preceding claims, wherein the at least one expansion body (10) has, in the state in which it is used, a proximal end (12₁₀), which is, in the state in which it is used, remote from a distal end (130) of the introduction device (120), and a distal end (14₁₀), which is, in the state in which it is used, facing the distal end (130) of the introduction device (120), and wherein at least the distal end (14₁₀) of the at least one expansion body (10) is blunt and/or designed especially with closed meshes (30, 30').

10. An introduction device according to any one of the preceding claims, wherein the at least one expansion body (10) has a coating (34), at least in a subarea (32), and/or wherein the coating (34) is a plastic film.

11. An introduction device according to any one of the preceding claims, comprising a proximal end (12), which is, in the state in which it is used, remote from a distal end (130) of the introduction device (120), and a distal end (14), which is, in the state in which it is used, facing the distal end (130) of the introduction device (120), the proximal end (12) and the distal end (14) being made of different materials.

## Revendications

1. Dispositif d'insertion (120) pour l'insertion d'un implant médical (102), lequel peut être libéré par un mouvement relatif entre un premier et un deuxième élément d'insertion (52 ; 54), comprenant un dispositif de recouvrement (100) pour le recouvrement d'un implant médical (102) partiellement libéré, comprenant au moins un corps d'expansion (10), sur lequel on peut régler une première extension radiale (16) et au moins une deuxième extension radiale (18), où la deuxième extension radiale (18) de l'au moins un corps d'expansion (10) peut être adaptée, lors du recouvrement de l'au moins un implant médical (102) partiellement libéré, à au moins une extension radiale (108) de l'au moins un implant médical (102) partiellement libéré, où l'au moins un corps d'expansion (10) peut être élargi de manière essentiellement homogène au moins dans au minimum une section axiale (20) dans la direction circonférentielle (22), où l'au moins un corps d'expansion (10) du dispositif de recouvrement (100) est posé radialement autour d'au moins un des éléments d'insertion (52, 54) et est disposé axialement mobile par rapport à moins un des éléments d'insertion (52, 54),
où le dispositif d'insertion (10) est conçu de telle manière que la libération de l'implant (100) commence à son extrémité distale (106),
où le premier élément d'insertion (52) est relié avec une pointe (140) du dispositif d'insertion (10) sous la forme d'un élément d'insertion interne,
où le deuxième élément d'insertion (54) est disposé radialement autour du premier élément d'insertion 52), interne sous la forme d'un élément d'insertion externe et n'est pas relié avec la pointe de cathéter (140) et est mobile en direction de l'extrémité proximale (125) du dispositif d'insertion (10) par rapport à la pointe de cathéter (140) par un mouvement axial de sorte qu'un implant (102) disposé radialement entre les éléments d'insertion interne et externe peut d'abord être libéré pour l'implantation avec son extrémité distale (106),
où le deuxième élément d'insertion (54) externe comprend un réceptacle pour l'implant, où l'implant est logé contre une paroi interne (56) radiale du deuxième élément d'insertion (54) de sorte que le deuxième élément d'insertion (54) agit en tant que corps d'immobilisation pour l'implant (102), **caractérisé en ce que** le dispositif de recouvrement est conçu sous la forme d'une gaine de stabilisation (36) du dispositif d'insertion (120) et
est posé radialement autour du deuxième élément d'insertion externe.

2. Dispositif d'insertion selon la revendication 1, dans lequel l'implant (102) est conçu sous la forme d'un implant (102) auto-expansible.

3. Dispositif selon la revendication 1 ou 2, dans lequel l'au moins un corps d'expansion (10) présente, au moins pour une deuxième extension (18) radiale réglée, une force de rappel agissant par rapport à un axe interne (135) du dispositif d'insertion (120) qui est prévue pour provoquer une nouvelle expansion de l'au moins un implant médical (102) au moins partiellement libéré.

4. Dispositif d'insertion selon l'une des revendications précédentes, dans lequel l'au moins une deuxième extension (18) radiale de l'au moins un corps d'expansion (10) est de préférence deux fois, de manière avantageuse trois fois, et de manière particulièrement préférée plus de cinq fois, plus grande par rapport à la première extension (16) radiale de l'au moins un corps d'expansion (10).

5. Dispositif d'insertion selon l'une des revendications précédentes, dans lequel l'au moins un corps d'expansion (10) présente au moins un tressage (24), lequel est conçu pliable de manière réversible, afin de régler la première extension (12) radiale et l'au moins une deuxième extension (18) radiale.

6. Dispositif d'insertion selon l'une des revendications précédentes, dans lequel l'au moins un corps d'expansion (10) présente un tressage (24), lequel est fabriqué à partir de fil rond (26) tressé entremêlé.

7. Dispositif d'insertion selon l'une des revendications précédentes, dans lequel l'au moins un corps d'expansion (10) est fabriqué en Elgiloy.

8. Dispositif d'insertion selon l'une des revendications précédentes, avec un dispositif de recouvrement comprenant une extrémité proximale (12), qui est éloignée à l'état d'utilisation d'une extrémité distale (130) du dispositif d'insertion (120), et une extrémité distale (14) qui est orientée à l'état d'utilisation vers l'extrémité distale (130) du dispositif d'insertion (120), où l'au moins un corps d'expansion (10) est disposé à l'extrémité distale (14) et/ou un segment faisant office de poignée (28) est disposé à l'extrémité proximale (12).

9. Dispositif d'insertion selon l'une des revendications précédentes, dans lequel l'au moins un corps d'expansion (10) dans l'état d'utilisation présente une extrémité proximale (12₁₀) qui est éloignée à l'état d'utilisation d'une extrémité distale (130) du dispositif d'insertion (120), et une extrémité distale (14₁₀) qui est orientée vers l'extrémité distale (130) du dispositif d'insertion(120) à l'état d'utilisation, et où au moins l'extrémité distale (14₁₀) de l'au moins un corps d'expansion (10) est conçu émoussée et/ou en particulier, avec des mailles fermées (30, 30').

10. Dispositif d'insertion selon l'une des revendications précédentes, dans lequel l'au moins un corps d'expansion (10) présente au moins un revêtement (34) sur une région partielle (32) et/ou le revêtement (34) est formé par un film en matière plastique.

11. Dispositif d'insertion selon l'une des revendications précédentes, comprenant une extrémité proximale (12) qui est éloignée d'une extrémité distale (130) du dispositif d'insertion (120) dans l'état d'utilisation, et une extrémité distale (14) qui est orientée vers l'extrémité distale (130) du dispositif d'insertion (120) à l'état d'utilisation, où l'extrémité proximale (12) et l'extrémité distale (14) sont fabriquées dans des matériaux différents.
